# EUROPEAN PATENT SPECIFICATION

(11) **EP 2 900 149 B1**
(45) Date of publication and mention of the grant of the patent: **09.05.2018**
(21) Application number: 13841823.1
(22) Date of filing: 30.08.2013
(51) Int. Cl.: A61B 17/04, A61B 17/17

(54) **TRANSOSSEOUS ATTACHMENT INSTRUMENTS**
TRANSOSSEALE BEFESTIGUNGSINSTRUMENTE
INSTRUMENTS DE FIXATION TRANS-OSSEUSE

(30) Priority: 28.09.2012 US 201261706921 P; 21.06.2013 US 201361837713 P
(43) Date of publication of application: 05.08.2015
(73) Proprietor: Tensor Surgical, Inc., Chattanooga, TN 37402 (US)
(72) Inventor: SANDERS, Brett, Signal Mountain, TN 37377 (US); HARPER, Keith, J., Chattanooga, TN 37405 (US)
(74) Representative: Santander Méndez, Patricia Victoria
(86) International application number: PCT/US2013/057485
(87) International publication number: WO 2014/051930

(56) References cited:
- WO-A2-2013/102909
- US-A- 5 630 824
- US-A1- 2010 121 375
- US-A1- 2010 312 249
- US-A1- 2010 312 249
- US-A1- 2013 123 809
- US-B1- 7 084 082
- US-B1- 7 247 164

## Description

### BACKGROUND OF THE INVENTION

This invention relates to techniques and instruments for surgical transosseous attachments.

This invention pertains to the attachment of, for example, ligaments, tendons, fascia, and muscle to an adjacent bone to effect a repair of a joint. Typical joints of a patient's body subjected to repair are the hands and feet, ankle, wrist, knee, elbow, hip, shoulder and spine. As a primary example, it is necessary to pass a suture through a portion of a bone to form a transosseous attachment to the bone. This invention is especially useful in arthroscopic surgical procedures for attaching a tendon of a rotator cuff muscle to the humerus bone for repairing a damaged shoulder joint, and is therefore described hereinafter with respect to such a procedure.

The tunneler instruments of the prior art are at a disadvantage in that they require excessive procedural steps to form the intersecting tunnels and transosseously pass suture through the intersecting tunnels. In addition, the prior art tunneler instruments cannot assure quick and effective transosseous placement and passing of the suture each and every attempt, and in addition, the prior art tunneler instruments are generally single use instruments which cannot be readily disassembled, disinfected and reused.

US2010/312249 relates to a drill guide and pin apparatus for creating bone tunnels. The apparatus comprises a drill guide and a pin. The drill guide has a drill sleeve and a boring tool operable to create a first bore in a bone. The pin is operably coupled to the boring tool by an arm and arranged to create a second bore in said bone at an angle of 30 to 170 degrees relative to the first bore. The first and second bores intersect at a predetermined position relative to the arm when the apparatus is in a drilling configuration. A catch is provided on the pin to allow a wise inserted through the first bore to be secured using surgical techniques.

### SUMMARY OF THE INVENTION

The present invention provides an apparatus for forming first and second transverse, intersecting bone tunnels and transosseously placing or passing a suture through the tunnels. A suture retriever spike having a window therethrough is inserted into one of the tunnels and aligned such that the window is aligned with the bore of the other of the tunnels. Suture is passed into the other tunnel with a suture passer and on through the spike window and there deposited. The spike is then removed from the one tunnel with the suture passed through the spike window, thereby transosseously retrieving the suture.

The first tunnel, normally a medial tunnel, may be formed by driving the suture retriever spike as an awl into the bone. The other or second tunnel, generally referred to as the lateral tunnel, may be created by driving a suture passer awl carrying suture into the bone and through the suture retriever spike window, thereby eliminating extra steps in the procedure by eliminating the need for first drilling the lateral tunnel. The suture passer awl is thereafter withdrawn while depositing the suture in the other or second tunnel, including the window of the suture retriever spike.

The tunneler instrument for accomplishing the method includes a guide handle having a proximal end for manual grasping and a distal end for engagement with the bone to which a suture is to be attached. The instrument further includes an independent suture retriever spike having a window therein, and the spike is received in the first or medial tunnel, preferably by driving the spike into the bone as an awl to thereby form the medial tunnel and minimize procedural steps.

A guide arm is carried at the distal end of the guide handle and the guide arm has a distal tip spaced from the outer surface of the distal end of the guide handle. The distal tip of the guide arm is configured for guided and oriented connection with the exposed proximal head of the suture retriever spike. This locks the suture retriever spike with the guide handle and thereby aligns the window of the spike with a central guide bore passing through the guide handle. This central bore is provided for receiving a bone tunneling implement to form a second or lateral tunnel in the bone which passes through the window of the implanted suture retriever spike.

The distal end of the guide handle is intended for engagement with the bone and is adjustably extendable relative to the remainder of the handle. This distal end of the handle is preferably provided with sharp teeth for securely engaging the bone surface. This adjustably extendable distal end of the guide handle is biased under spring pressure in or to a forward position or direction relative to the remainder of the guide handle. Thus once the suture retriever spike is engaged with the guide arm, the forward bone engaging tip of the distal end of the guide handle snugly engages the bone's surface to prevent slippage, and it is assured that the handle guide bore is aligned with the window of the suture retriever spike.

The guide arm secured to the spike head is pivotally connected to the forward end of the guide handle so that the guide arm may pivot in a plane which incorporates the transverse intersecting axis of the spike and the axis of central guide bore of the handle, or in the plane of the first and second or medial and lateral tunnels. This permits the surgeon to position the lateral tunnel at a desired angle with respect to the medial tunnel as desired, yet insures that the lateral tunnel will properly intersect with the medial tunnel and the suture retriever window.

To assist in retaining the passed suture in the suture retriever spike window, a suture engaging device or membrane may be provided and retained in the spike window. This ensures engagement and retention of the suture within the spike window as the suture passer is withdrawn from the spike window.

### BRIEF DESCRIPTION OF THE DRAWINGS

Other objects and advantages appear hereinafter in the following description and claims. The accompanying drawings show, for the purpose of exemplification, without limiting the scope of the present invention and the appended claims, certain practical embodiments of the invention wherein:

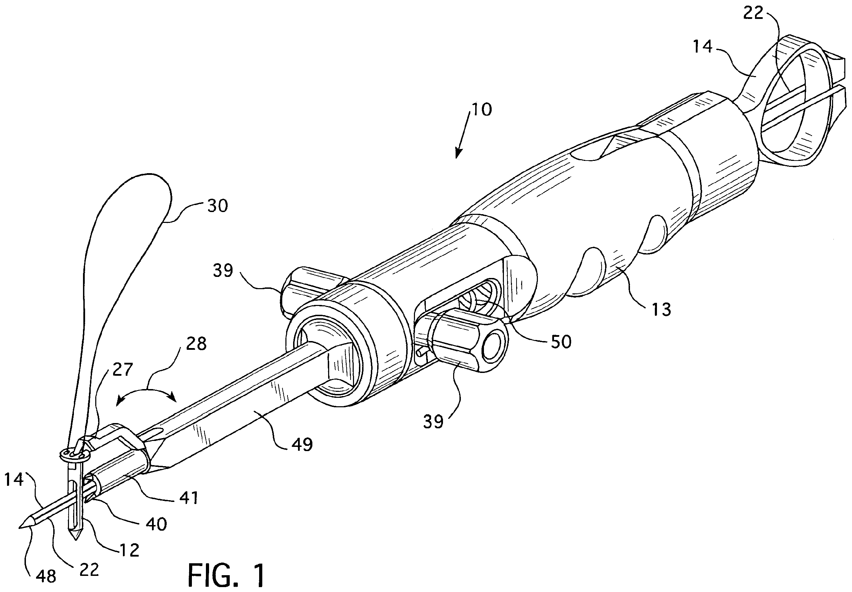

FIG. 1 is a perspective view of a set of instruments which comprise the tunneler according to the present invention;
FIG. 2 is an enlarged perspective view of the forward tip portion of the tunneler, illustrated in FIG. 1 without the inclusion of the suture retriever spike and the suture pusher awl instruments;
FIG. 3 is an enlarged perspective view of the forward tip portion of the tunneler instrument assembly shown in FIG. 1;

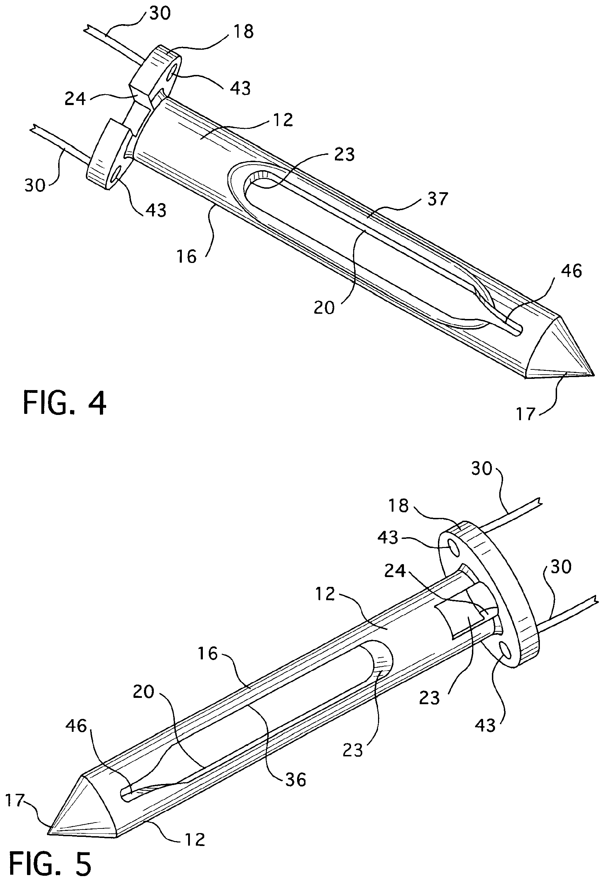

FIG. 4 is an enlarged perspective view of the rearward face of the suture retriever spike instrument utilized in the combination shown in FIG. 1;
FIG. 5 is an enlarged perspective view illustrating the forward face of the suture retriever spike instrument shown in FIG. 4;
FIG. 6 is an enlarged top view of the suture retriever spike instrument shown in FIG. 4;
FIG. 7 is a perspective view of the driver instrument utilized to drive and insert the attached suture retriever spike shown in FIG. 4 into bone to create a first or medial tunnel;
FIG. 8 is an enlarged perspective view of the forward end of the driver instrument shown in FIG. 7 with the suture retriever spike instrument shown in FIG. 4 attached to the forward end thereof;
FIG. 9 is an enlarged perspective of the forward tip portion of the driver instrument shown in FIG. 7 without the suture retriever spike attached;
FIG.10 is a perspective view of the suture pusher awl instrument utilized in the combination shown in FIG. 1;
FIG. 11 is an enlarged perspective view of the forward tip portion of the suture pusher awl instrument shown in FIG. 10;
FIG. 12 is an enlarged perspective view of the forward tip portion of the suture pusher awl of FIG. 10 shown from the bottom side thereof and with the suture removed; and
FIG. 13 is a side view in partial section illustrating the application of the instruments of the present invention to a humeral head.

### DETAILED DESCRIPTION OF THE DRAWINGS

The following illustrated example depicts instruments and techniques to pass a suture through a portion of the head of the humeral bone at the shoulder of a human patient to repair damaged soft tissue associated with the shoulder joint. However, instruments and techniques according to the present invention may be used to pass a suture through any bone, at surgical sites anywhere in a patient's body, and for any purpose.

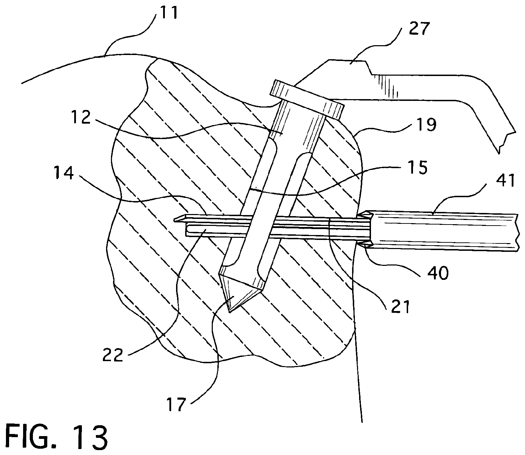

Referring to the drawings, the tunneler instrument 10 is a combination of instruments used for the creation of converging tunnels in bone (transosseous tunneling), both arthroscopically or with open techniques. For illustration purposes only, the tunneler 10 of the present invention is illustrated in the creation of tunnels in the humeral head 11 as shown in FIG. 13 to allow for the repair of a torn rotator cuff.

The tunneler instrument 10 consists of three primary components, a suture retriever spike 12 (which serves as a guide reference for the tunneler instrument 10), a guide handle 13, and a suture pusher awl 14. These three instruments work together to create two converging tunnels of different length and angles within bone and transosseously pass suture through the tunnels, with fewer steps and greater reliability and repeatability than is possible through the use of current techniques.

The suture retriever spike 12 is used to create the first or medial tunnel 15 (FIG. 13) in the humeral head 11. The suture retriever spike 12 is comprised of a rigid cylindrical body 16 constructed of surgical stainless steel or other suitable material, with a sharp distal tip 17 provided for piercing bone as an awl, and a flat proximal head 18 meant to sit on the outer surface 19 of bone 11. A window 20 is provided in the mid section of spike 12 to allow passage of the complimentary suture pusher awl 14, which is used to create the second converging lateral tunnel 21, as well as for passing suture 22 through the lateral tunnel.

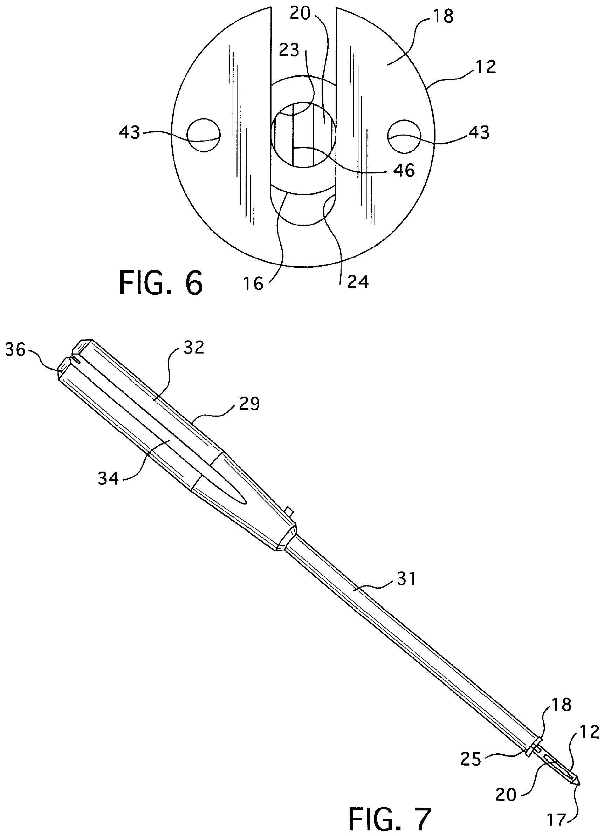

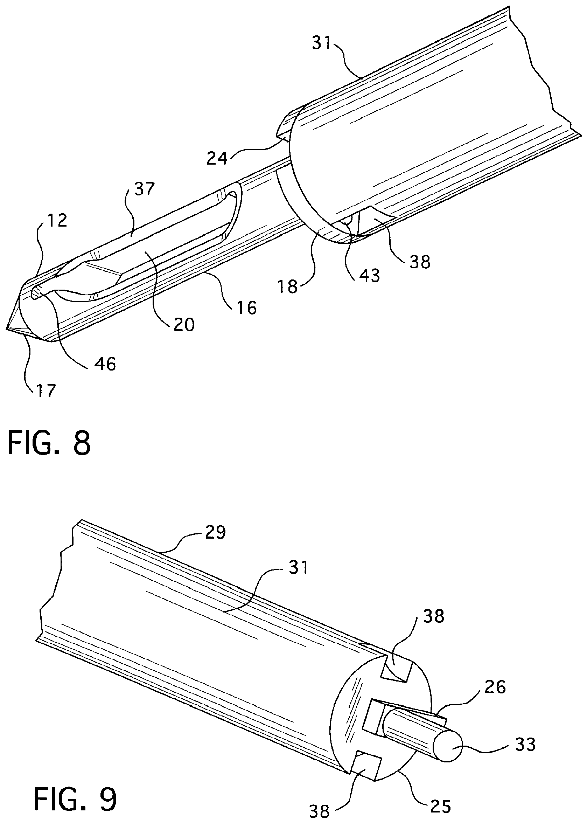

The head 18 of suture retriever spike 12 is cannulated axially as indicated at 23 and slotted radially at 24 to allow for mounting of the spike 12 onto the distal connecting end 25 of driver 29 (FIGS. 7, 8 and 9) for driving spike 12 into the bone 11. A tooth 26 is provided on the distal connecting end 25 of driver 29 which cooperatively fits into the slot 24 of spike 12 to prevent rotation of the spike 12 relative to the driver 29. The same slot 24 also, after the suture retriever spike 12 is imbedded in bone 11, receives the rigid guide arm 27 of guide handle 13 and thereby orients and aligns the spike 12 as desired and prevents yaw and roll of the guide handle 13 relative to spike 12, but allows for vertical pivotal rotation or pitch of the guide handle relative to the spike as indicated by arrow 28. This coupling arrangement between guide arm 27 and spike 12 also aligns the window 20 of spike 12 with the axis of the guide bore 42 for receiving suture pusher awl 14 in guide handle 13 so that the tip of suture pusher awl 14 aligns with and will pass through window 20 of spike 12.

A long loop 30 of suture or wire is secured via passages 43 to head 18 of spike 12 and permits the surgeon or assistant to pull the spike 12 out of the bone 11 when desired to retrieve suture 22. The end of this loop 30 stays well outside of the body at all times, allowing the surgeon or assistant to readily grab it with their hand and pull.

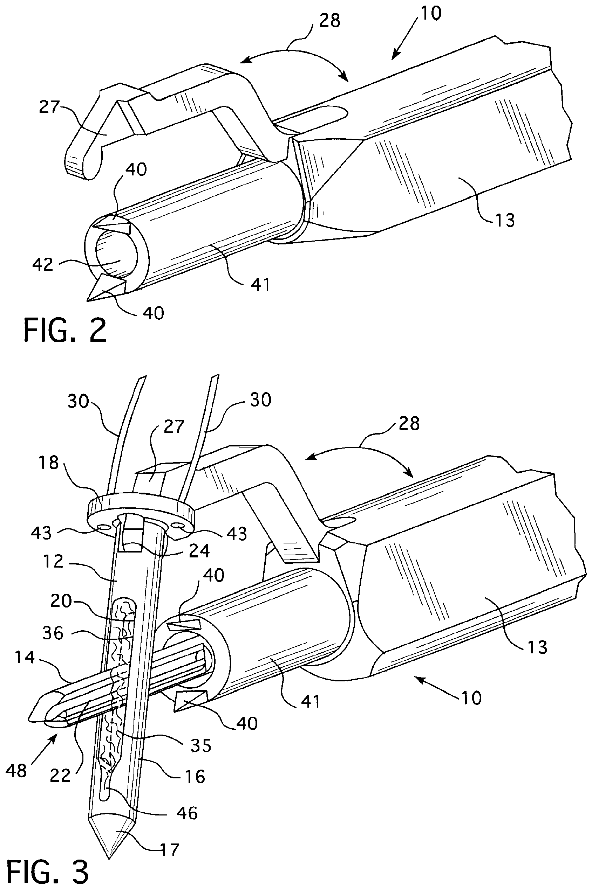

The window 20 in the middle section of spike 12 is filled with a suture engaging membrane 35 (FIG. 3) which may be silicone or a similar semi-solid substance, or alternatively may be a mechanical device such as a spring or trap door which permits the spike 12 to actively grasp suture 22 that is passed by suture pusher awl 14 through the window 20. Such a trapdoor would consist of two or more hinged door elements which are spring loaded to engage the sides of the forward shaft of suture pusher 14, and to thereby engage and grip suture 22 on the exterior side surfaces of the pusher shaft, so that when the pusher shaft of suture pusher 14 is withdrawn from lateral tunnel 21, suture 22 is forced to remain within lateral tunnel 21.

The membrane 35 in the form of silicone, in a manner similar to the aforedescribed mechanical trap door, compresses against the shaft of suture pusher awl 14 and engages suture 22 with resistance while permitting the smooth surfaces of the shaft portion of suture pusher 14 to be withdrawn from window 20 of spike 12. As another alternative to a semisolid substance, such as silicone, membrane 35 may be a pierceable sheet or membrane of plastic or metal, retained within window 20 of spike 12, so that the edges of the puncture opening made by the suture pusher awl 14 in the semirigid sheet or membrane engage and retain the suture 22 as the shaft of suture pusher awl 14 is withdrawn from the membrane or sheet through window 20.

Returning to FIGS. 7, 8 and 9, the spike driver 29 consists of a shaft 31 with a handle 32 on the proximal end and a small round post 33 and tooth 26 on its distal end. A slot 34 is provided on handle 32 to grip the suture or wire loop 30 attached to spike head 18, thereby preventing the spike from falling off of the tip of the driver 26 after the spike 12 has been positioned on the tip 25. Relief slots 38 on the forward end of shaft 31 permit egress of suture 22 leading from passages 43 of spike head 18. A mallet is then used on the distal end 36 of driver 26 to drive spike 12 into the bone 11 until the head 18 is flush with the bone's surface 19 (see FIG. 13). At this point the retrieval loop 30 of wire or suture is removed from the slot 34 of the handle 32 of driver 29, and the driver 29 is removed from the spike 12, leaving the spike 12 in the bone 11 to act as both a reference point for the guide handle 13 and for suture capture and passing of a tunneling mechanism and suture 22 through the second or lateral tunnel 21.

After spike 12 is set, the guide handle 13 is then used to create the second transverse converging lateral tunnel 21. The tip of inner guide 41 of guide handle 13 is provided with teeth 40 to securely engage the bone 11. Inner guide 41 is cannulated to provide guide bore 42 to allow for passage of a drill bit, an awl and/or a suture pusher, or any combination thereof. The tip of guide arm 27 of guide handle 13 is precisely machined to fit snugly into the slot 24 of the head 18 of spike 12, thereby preventing yaw and roll of the guide handle 13 relative to the implanted spike 12, but allowing for free pivoting or pitch of the guide arm 27 as indicated by arrow 28. This restriction of motion along two axes, but free motion along the third axis, allows the user to place the converging lateral tunnel 21 anywhere within the "range" of the guide handle 13 along the aspect of the bone surface 19. The pivoting of guide arm 27 allows for the second or lateral tunnel 21 to have an entry point on surface 19 closer to or farther from the head of spike 12 as desired, and concomitantly provides a more acute or obtuse angle of the second lateral tunnel 21 relative to the first medial tunnel 15. When the guide arm 27 is engaged in the head 18 of spike 12, the guide handle 13 is able to rotate the spike 12 within its tunnel 15 to change the lateral position of the entry point of the second tunnel 21 relative to the spike 12, while still maintaining and ensuring alignment of the guide bore 42 with the spike window 20.

The suture pusher awl 14 is preloaded with suture 22 so that one can deliver mallet strikes to the proximal end of pusher awl 14 and thereby deliver suture and punch out lateral tunnel 21 at the same time without drilling.

After the desired position of the second or lateral tunnel 21 is achieved, the user is able to advance the inner guide 41 toward the bone under the forward bias of compression spring 34, thereby engaging the teeth 40 on the distal tip of inner guide 41 into the bone, thus "setting" the position of the second tunnel 21. Handles 39 are utilized to pull back the tip of inner guide 41 against the forward urging of spring 50 to adjust the position of inner guide 41. After the position is set, the second tunnel is created by driving the suture passer awl 14, preloaded with the endless passing suture loop 22, after sliding the combination through the guide bore 42 of the inner guide 41, and driving awl 14 into the bone and through the window 20 of spike 12. The suture pusher awl 14 is then removed, but the passing suture 22 is left behind in the bone tunnel 21 and extending through the window 20 of the spike 12.

To further assist in ensuring that suture 22 is left behind in window 20 of spike 12, the width of window 12 is dimensioned for a tight fit whereby the side walls of window 20 will slidingly engage suture 22 as suture passer awl 14 is passed through into window 20. Upon removal of suture passer awl 14, the sharp rear edges 36 of window 20 will engage suture 22 as another example of the suture engaging device to ensure that it slips off of suture pusher awl 14 as it is removed such that the suture 22 remains within the passage of window 20 of spike 12. To further assist in ensuring that the forward tip of suture pusher awl 14 will find the opening of spike window 20, a beveled edge 37 is provided about the perimeter edge of window 20. The bottom of spike window 20 is provided with a narrowing passage 46 so that when spike 12 is withdrawn, the suture 22 will be wedged into and thereby retained in passage 46.

If the bone 11 is too hard for use of the pusher awl 14, a backup drill bit or standard awl can be used to create the second tunnel 21 through which a passing suture can then be pushed.

After the suture 22 is passed through the window 20 of spike 12, suture pusher awl 14 is withdrawn leaving suture 22 behind and the guide handle 13 is then removed from the spike 12 and the remaining suture 22, so that only the suture 22 and the spike 12 remain in the bone 11. The retrieval loop 30 of spike 12 is then pulled, removing the spike and the passing loop of suture 22 from the first tunnel 15, thereby allowing the passing loop of suture 22 to be used to pass any number of functional sutures through the completed tunnel configuration.

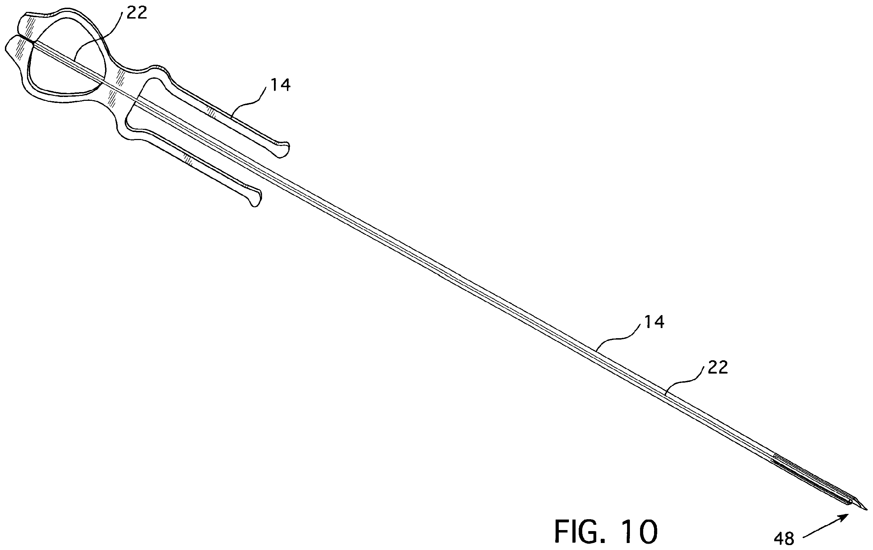

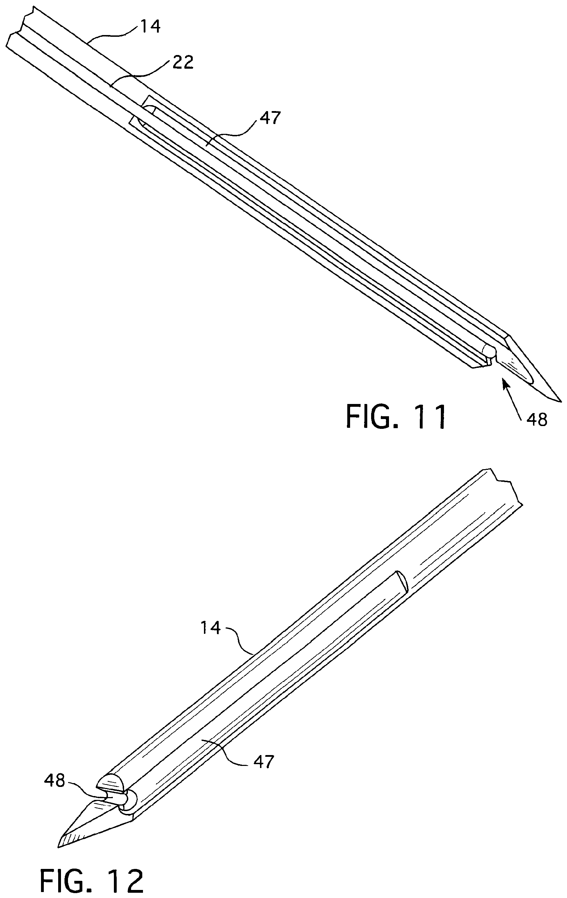

The suture pusher awl 14 has a special "shark mouth" geometry such that a slot 48 is created in the distal tip (see FIGS. 10, 11 and 12), allowing it to passively capture the suture 22 in slot 48 and side grooves 47 while creating the tunnel, but release the suture 22 as it is withdrawn from the tunnel 21.

The tunneler instrument 10 of the present invention accordingly provides a highly accurate guidance system insuring that the second intersecting lateral tunnel 21 is accurately aligned with the first medial tunnel 15 and carries out the procedure with less steps than required by the instruments of the prior art and also assures complete suture passage on the first attempt.

All of the instrument parts of the tunneler instrument 10 may be disassembled in large sections so that the assembly may be readily broken down, sterilized, and reused and reassembled easily by hand. For example, inner guide 41 unscrews from outer guide 49 and outer guide 49 in turn may be threadably uncoupled in a conventional manner from the handle portion of guide handle 13. Also, handles 39 may be unscrewed and removed so that spring 50 and its associated assembly may be removed, along with inner guide 41 and outer guide 49 for sterilization.

## Claims

1. A medical instrument for placing a suture transosseously through first and second transverse, intersecting bone tunnels, the instrument including in combination:
a guide handle (13) having an elongate proximal end for manual grasping and a forward distal end for engagement with a surface of a bone (11) to which a suture (22) is to be attached, said bone (11) provided with a first tunnel (15);
a suture retriever spike (12) having a window (20) therethrough, said spike (12) dimensioned for reception in said first tunnel (15), and
a guide arm (27) carried at the distal end of said guide handle (13) forward of said elongate proximal end but aft of said guide handle (13) distal end and having a distal tip spaced from an outer surface of said distal end and connected to a proximal head of said spike (12), the combined length and orientation of said spike (12) and said guide arm (27) relative to said guide handle (13) being prefixed for thereby aligning said window (20) with a guide bore (42) passing longitudinally through said handle (13) and receiving a bone tunneling implement to form a second tunnel (21) in the bone which passes through said window (20);
**characterized in that** said bone tunneling implement is a suture passer in the form of a suture pusher awl (14) having an elongate awl shaft with a sharp point at its distal tip for forming said second tunnel (21), said pointed awl shaft having a forward facing suture receiving mouth at its distal tip just aft of said point for receiving and advancing a loop of suture (30) with said awl shaft while forming said second tunnel (21) through said spike window (20) for deposit of the suture loop (30) in said spike window (20) upon withdraw of said awl shaft from said second tunnel (21).

2. The medical instrument of claim 1, wherein said distal end of said guide handle (13) is adjustably extendable relative to the remainder of said handle (13).

3. The medical instrument of claim 2, wherein said distal end of said guide handle (13) has at least one sharp tooth (40) for engaging a bone surface (19).

4. The medical instrument of claim 2, wherein said adjustably extendable distal end of said guide handle (13) is biased in a forward direction relative to said guide handle (13).

5. The medical instrument of claim 1, wherein said guide arm (27) is pivotally connected to said guide handle (13) to pivot in a plane incorporating the transverse intersecting axes of said spike (12) and said guide bore (42).

6. The medical instrument of claim 1, wherein said passive suture engaging device is a suture engaging membrane (35).

7. The medical instrument of claim 1, wherein said spike window (20) is dimensioned in width to engage suture exposed on side surfaces of a suture passer to assist in retaining said suture (22) in said window (20) as said suture passer is withdrawn from said window (20).

8. The medical instrument of claim 7, wherein said spike window (20) is provided with a beveled face edge (37) on the side of said spike window (20) facing said second tunnel (21).

9. The medical instrument of claim 1, wherein said spike window (20) includes a narrowing recess (46) at the bottom thereof for wedgingly engaging suture (22) placed in said spike window (20).

10. The medical instrument of claim 1, wherein the distal tip of said guide arm (27) is detachable from said spike (12).

## Patentansprüche

1. Ein medizinisches Instrument zur transossealen Platzierung eines Nahtmaterials durch einen ersten und einen transversal dazu verlaufenden und diesen schneidenden zweiten Knochentunnel, wobei das Instrument eine Kombination der folgenden Elemente einschließt:
einen Führungsgriff (13) mit einem langgestreckten proximalen Ende zum manuellen Festhalten und ein vorderes distales Ende zum Eingreifen in eine Oberfläche eines Knochens (11), an dem ein Nahtmaterial (22) befestigt werden soll, wobei der Knochen (11) mit einem ersten Tunnel (15) versehen ist;
einen Nahtmaterial-Rückziehdorn (12) mit einem durch diesen hindurchreichenden Fenster (20), wobei der Dorn (12) zur Aufnahme in den ersten Tunnel (15) dimensioniert ist, und
einen Führungsarm (27), der sich am distalen Ende des Führungsgriffs (13) vor dem langgestreckten proximalen Ende, aber hinter dem distalen Ende des Führungsgriffs (13) befindet und eine distale Spitze hat, die zur Außenseite des distalen Endes beabstandet ist und mit einem proximalen Kopf des Dorns (12) verbunden ist, wobei die kombinierte Länge und Ausrichtung des Dorns (12) und des Führungsarms (27) relativ zum Führungsgriff (13) vorgegeben sind, so dass das Fenster (20) mit einer Führungsbohrung (42) ausgerichtet wird, die längs durch den Griff (13) verläuft und ein Knochentunnelinstrument aufnimmt, um einen zweiten Tunnel (21) in dem Knochen herzustellen, der durch das Fenster (20) verläuft;
**dadurch gekennzeichnet, dass** das Knochentunnelinstrument ein Nahtführungsinstrument in Form eines Nahtvorschubpfriems (14) ist, der einen langgestreckten Pfriemschaft mit einer scharfen Spitze an seinem Ende zur Formung des zweiten Tunnels (21) hat, wobei der spitze Pfriemschaft an seiner distalen Spitze direkt hinter der scharfen Spitze eine nach vorne gerichtete, das Nahtmaterial aufnehmende Öffnung zur Aufnahme und Weiterführung einer Nahtmaterialschlaufe (30) mit dem Pfriemschaft hat, während der zweite Tunnel (21) durch das Dornfenster (20) hergestellt wird, so dass die Nahtmaterialschlaufe (30) nach dem Herausziehen des Pfriemschafts aus dem zweiten Tunnel (21) in dem Domfenster (20) abgelegt wird.

2. Das medizinische Instrument nach Anspruch 1, wobei das distale Ende des Führungsgriffs (13) relativ zum restlichen Teil des Griffs (13) verstellbar ausziehbar ist.

3. Das medizinische Instrument nach Anspruch 2, wobei das distale Ende des Führungsgriffs (13) mindestens einen scharfkantigen Zahn (40) zum Eingriff in eine Knochenoberfläche (19) hat.

4. Das medizinische Instrument nach Anspruch 2, wobei das verstellbar ausziehbare distale Ende des Führungsgriffs (13) relativ zu dem Führungsgriff (13) in Vorwärtsrichtung gespannt ist.

5. Das medizinische Instrument nach Anspruch 1, wobei der Führungsarm (27) schwenkbar mit dem Führungsgriff (13) verbunden ist, so dass er in einer Ebene geschwenkt werden kann, die die transversalen, sich schneidenden Achsen des Dorns (12) und der Führungsbohrung (42) einschließt.

6. Das medizinische Instrument nach Anspruch 1, wobei die passive Nahtmaterial-Haltevorrichtung eine Nahtmaterial-Haltemembran (35) ist.

7. Das medizinische Instrument nach Anspruch 1, wobei das Domfenster (20) in seiner Breite so dimensioniert ist, dass es das an den Seitenflächen eines Nahtführungsinstruments freiliegende Nahtmaterial festhält, um dazu beizutragen, das Nahtmaterial (22) in dem Fenster (20) zu halten, während das Nahtführungsinstrument aus dem Fenster (20) herausgezogen wird.

8. Das medizinische Instrument nach Anspruch 7, wobei das Domfenster (20) an der Seite des Dornfensters (20), die zum zweiten Tunnel (21) hin gerichtet ist, mit einer abgeschrägten Kante (37) versehen ist.

9. Das medizinische Instrument nach Anspruch 1, wobei das Domfenster (20) an seiner Unterseite eine sich verjüngende Aussparung (46) hat, in der das in das Dornfenster (20) platzierte Nahtmaterial (22) verkeilend festgehalten wird.

10. Das medizinische Instrument nach Anspruch 1, wobei die distale Spitze des Führungsarms (27) von dem Dorn (12) trennbar ist.

## Revendications

1. Instrument médical destiné à placer un fil de suture de manière trans-osseuse à travers une première et une seconde transversale, croisant des tunnels osseux, l'instrument comprenant en association :
une poignée de guidage (13) ayant une extrémité proximale allongée pour une préhension manuelle et une extrémité distale avant destinée à entrer en contact avec une surface d'un os (11) auquel un fil de suture (22) doit être fixé, ledit os (11) étant doté d'un premier tunnel (15) ;
une pointe (12) de récupération de fil de suture ayant une fenêtre (20) en son sein, ladite pointe (12) étant dimensionnée pour recevoir dans ledit premier tunnel (15), et
un bras de guidage (27) porté au niveau de l'extrémité distale de ladite poignée de guidage (13) à l'avant de ladite extrémité proximale allongée mais à l'arrière de ladite extrémité distale de poignée de guidage (13) et ayant une extrémité distale éloignée d'une surface extérieure de ladite extrémité distale et reliée à une tête proximale de ladite pointe (12), la longue et l'orientation combinées de ladite pointe (12) et dudit bras de guidage (27) par rapport à ladite poignée de guidage (13) étant fixées au préalable afin de pouvoir ainsi aligner ladite fenêtre (20) avec un trou de guidage (42) traversant longitudinalement ladite poignée (13) et
recevoir un accessoire de tunnellisation d'os pour former un second tunnel (21) dans l'os qui traverse ladite fenêtre (20) ; **caractérisé en ce que** ledit accessoire de tunnellisation d'os est un dispositif de passage de fil de suture sous la forme d'une alêne (14) de poussoir de fil de suture ayant une tige d'alêne allongée dotée d'une pointe coupante à son extrémité distale destinée à former ledit second tunnel (21), ladite tige d'alêne pointue ayant un bec de réception de fil de suture faisant face vers l'avant au niveau de son extrémité distale juste après ladite pointe destinée à recevoir et à faire avancer une boucle de fil de suture (30) avec ladite tige d'alêne formant ledit second tunnel (21) à travers ladite fenêtre (20) de pointe pour déposer la boucle (30) de fil de suture dans ladite fenêtre (20) de pointe en retirant ladite tige d'alêne dudit second tunnel (21).

2. Instrument médical selon la revendication 1, ladite extrémité distale de ladite poignée (13) de guidage étant extensible de manière réglable par rapport au reste de ladite poignée (13).

3. Instrument médical selon la revendication 2, ladite extrémité distale de ladite poignée (13) de guidage ayant au moins une dent coupante (40) destinée en entrer dans une surface osseuse (19).

4. Instrument médical selon la revendication 2, ladite extrémité distale extensible de manière réglable de ladite poignée de guidage (13) est sollicitée dans une direction vers l'avant par rapport à ladite poignée de guidage (13).

5. Instrument médical selon la revendication 1, ledit bras de guidage (27) est relié pivotant à ladite poignée de guidage (13) pour pivoter dans un plan incorporant les axes croisant la transversale de ladite pointe (12) et dudit trou de guidage (42).

6. Instrument médical selon la revendication 1, ledit dispositif d'insertion de fil de suture passif est une membrane d'insertion (35) de fil de suture.

7. Instrument médical selon la revendication 1, ladite fenêtre (20) de pointe est dimensionnée en largeur pour engager le fil de suture rendu visible sur les surfaces latérales d'un dispositif de passage de fil de suture pour aider à retenir ledit fil de suture (22) dans ladite fenêtre (20) au moment où ledit dispositif de passage de fil de suture est retiré de ladite fenêtre (20).

8. Instrument médical selon la revendication 7, ladite fenêtre (20) de pointe est dotée d'un bord (37) à face biseautée sur le côté de ladite fenêtre (20) de pointe faisant face au dit second tunnel (21).

9. Instrument médical selon la revendication 1, ladite fenêtre (20) de pointe comprenant un évidement (46) se rétrécissant dans son fond pour caler le fil de suture dans ladite fenêtre (20) de pointe.

10. Instrument médical selon la revendication 1, ladite extrémité distale dudit bras (27) de guidage peut se retirer de ladite pointe (12).
